# EUROPEAN PATENT SPECIFICATION

(11) **EP 2 155 161 B1**
(45) Date of publication and mention of the grant of the patent: **28.12.2016**
(21) Application number: 08737302.3
(22) Date of filing: 14.03.2008
(51) Int. Cl.: A61K 47/02, A61K 47/14, A61K 47/18, A61K 47/40

(54) **STABLE LIQUID COMPOSITION OF CHONDROITINSULFATE AND GLUCOSAMINE**
STABILE FLÜSSIGE ZUSAMMENSETZUNG VON CHONDROITINSULFAT UND GLUCOSAMIN
COMPOSITION LIQUIDE STABLE DE SULFATE DE CHONDROÏTINE ET DE GLUCOSAMINE

(30) Priority: 01.06.2007 IT MI20071120
(43) Date of publication of application: 24.02.2010
(73) Proprietor: Apharm S.r.l., 28041 Arona (NO) (IT)
(72) Inventor: PIZZONI, Angelo, I-28041 Arona (NO) (IT)
(74) Representative: Trupiano, Federica
(86) International application number: PCT/IB2008/000595
(87) International publication number: WO 2008/146104

(56) References cited:
- EP-A- 0 704 216
- EP-A1- 1 681 065
- WO-A-01/93847
- JP-A- 1 016 728
- US-A1- 2002 032 171
- US-B1- 6 979 458

## Description

### FIELD OF THE INVENTION

The present invention concerns a stable liquid composition comprising a glycosaminoglycan and N-acetylated glucosamine and a cyclodextrin for oral administration. More particularly, it is an object of the present invention to provide a formulation consisting of a solution containing, as active ingredients thereof, chondroitinsulfate and glucosamine, useful for the prevention or of the slowdown of arthrosis, with a higher stability in respect of formulation already existing on the market.

### BACKGROUND OF THE INVENTION

Glycosaminoglycans are a family of polysaccharides each formed by a mixture of chains wherein each chain is a compound consisting of a repetitive sequence of a uronic acid-hexosamine disaccaride, said uronic acid unit, glucuronic acid or iduronic acid, being linked in α 1→4 or β 1→3 to said hexosamine unit, glucosamine or galactosamine, and said uronic acid and hexosamine units being sulfated at different extent.

Beside heparin and heparan sulfate, also dermatan sulfate, hyaluronic acid and chondroitinsulfate belong to the glycosaminoglycans' family. Physiologically, glycosaminoglycans are organized in proteoglycans formed by a protein core wherein the glycosaminoglycan is linked by a linkage region. These structures are in charge of the control of biochemical reactions by the uptake and the release of proteins and growth factors (J.F. Kennedy, C. A. White, Bioactive Carbohydrates, 1983, Ellis Horwood Ltd, 211-227).

In particular, chondroitinsulfate is located in cartilages to give them elasticity and to control their resistance. In addition, it has an inhibiting effect on collagenase and elastase, which are cartilage depolymerizing enzymes. In a healthy subject, the cartilage destruction and reconstruction processes are in equilibrium but, when the cartilage destruction process is more rapid than the reconstruction one, a pathological condition occurs, for example arthrosis or arthritis.

In nature, chondroitinsulfate exists in two structural forms: chondroitin-4-sulfate (or ChSA) and chondroitin-6-sulfate (or ChSC). It consists of a mixture of chains wherein each chain is a compound (molecule) showing a repetitive sequence of a glucuronic acid-galactosamine disaccharide wherein the glucuronic acid is linked to galactosamine by a β 1→3 bond, said galactosamine being 4-O-sulfated (ChSA) or 6-O-sulfated (ChSC). In the same molecule, clusters of ChSA and ChSC coexist. The proteoglycans containing chondroitinsulfate are formed by a hyaluronic acid skeleton onto which protein chains (protein core) bearing branches of condroitisulfate chains are grafted. A highly viscous macromolecule is thus formed whose viscosity is due to the very high molecular mass and also to the structural characteristics of its components.

Glucosamine is the monosaccharide starter of the biosynthesis of the glycosaminoglycans and, hence, its presence in a high amount shifts the equilibrium of the cartilage metabolism toward the biosynthesis.

Unless otherwise specified, in the present description the term "chondroitinsulfate" designates chondroitinsulfate in sodium salt form or of another of its salts, the dosages given herein below being referred to sodium chondroitinsulfate. The term "glucosamine" designates the D-glucosamine (2-amino-2-deoxy-D-glucose), as such or in form of an acid addition salt thereof, the dosages given herein below being referred to glucosamine base.

### PRIOR ART

It is known that chondroitinsulfate is used in various forms as integrator in the prevention of arthrosis and in the treatment of different pathologic conditions of cartilage joints, such as inflammatory processes and osteoarthrosis, as described for example in EP 704216.

Pharmaceutical or "nutraceutical" compositions containing chondroitinsulfate in association with glucosamine for their use in the protection and reparation of connective tissue are described in US 5,364,845 and US 5,587,363.

Pharmaceutical forms for the oral administration of chondroitinsulfate, in tablets, capsules granules and solutions are already existing on the market. Among said forms, the solution is the most immediately available additive for the oral administration in the prevention of arthrosis. However, an aqueous solution of chondroitinsulfate tends to turn yellow, even in the presence of preservatives. As a consequence, a pharmaceutical or "nutraceutical" composition in form of an aqueous solution of chondroitinsulfate becomes unpleasant during the time, even though the loss of activity is not appreciable.

Thus, there is a need of avoiding the coloring of such solutions, which are in form of a gel and whose viscosity is due to the macromolecular nature of chondroitinsulfate, in order to improve their organoleptic character and to assure the maximal activity of all of their components at the moment of use.

Glucosamine too, as free base or in form of one of its salts such as the hydrochloride or the sulfate, is not very stable in aqueous solution and tends to give a yellow coloration very probably due to oxidation products.

In order to avoid the formation of such oxidation products and to keep the formation of moulds and bacteria under control, known preservatives such as sodium benzoate, parabens, ethylenediaminotetracetic acid (EDTA), as such or as sodium salt, or potassium sorbate are added to the oral liquid preparation of chondroitinsulfate and/or glucosamine.

Moreover, in order to avoid the oxidative degradation, antioxidants such as sodium metabisulfite or sodium sulfite are added to aqueous solutions containig chondroitinsulfate and/or glucosamine.

However, all these substances do not allow an effective conservation of the solutions during the time and the inhibition of the formation of the yellow color.

The above-mentioned EP 704216 document, for example, discloses a gel formulation containing 1.2 g of an organic or inorganic chondroitinsulfate salt in admixture with potassium sorbate and sodium benzoate as preservatives. Among the organic salts, the glucosamine salt is also mentioned. It has been observed that the above-mentioned preservatives in such a composition do not preserve the components from oxidation, all the more in the presence of glucosamine.

The use of cyclodextrins, for example α-cyclodextrin, β-cyclodextrin, γ-cyclodextrin, hydroxypropyl-β-cyclodextrin, hydroxypropyl-γ-cyclodextrin for solubilizing compounds which are not water soluble or for avoiding incompatibility between the various components of a solution is well known in the literature.

Document US 2005/0256083 discloses a pharmaceutical ophthalmic composition comprising a cyclodextrin, a guanidine cationic compound and sorbic acid. In addition, it cites a series of documents wherein the use of cyclodextrins is reviewed.

Among these cited documents, in particular, JP 01-16728 discloses an ophthalmic aqueous solution containing a drug, for example sodium hyaluronate, chondroitinsulfate or lysozyme chloride, an antiseptic cationic surface-active agent, such as benzalconium chloride or chlorhexidine hydrochloride, and a cyclodextrin or a derivative thereof. In this case, the cyclodextrin serves to inhibit clouding and precipitation. US 6979458 B1 discloses aqueous oral wellness dosage form with glucosamine and chondroitin sulfate, and higher concentrations of vitamin C and Vitamin E. But no cyclodextrin is present.

JPS6416728A describes clear eye drops not becoming cloudy comprising chondroitin sulfate, cationic surface active agent and cyclodextrin. no N-acetylated glucosamine and no antioxidant is present.

### SUMMARY OF THE INVENTION

It has now been found that the addition of a cyclodextrin to a solution of chondroitinsulfate, of glucosamine, of antioxidants such as sodium metabisulfite and of preservatives such as sodium benzoate or potassium sorbate allows the concurrent presence of the two active principles, of the antioxidants and of the preservatives in a solution which does not tend to yellow and that remains intact for a longer time.

In particular, it has been found that cyclodextrins complex for example sodium benzoate (as described by SIIMER E. et al. Thermochimica acta , 1987, vol. 116, pp. 249-256), which is a preferred preservative, and concurrently also favorably interact with glucosamine by including it in their skeleton (as also described by SAKAIRI N. et al. J. Chem. Soc. Chem. Commun, 1991, n. 5, pp. 289-290).

### DETAILED DESCRIPTION

It is an object of the present invention to provide a composition for oral administration in form of an aqueous solution which comprises
(a) chondroitinsulfate at a concentration of from 5% to 15% and N-acetylated glucosamine at a concentration of from 6% to 20%;
(b) a cyclodextrin, at a concentration of from 4% to 10%;
(c) antioxidants at a total concentration of from 0.2 to 0.4%;
(d) preservatives at a total concentration of from 0.1% to 0.3%.

In the present formulation, the chondroitinsulfate will be in form of an alkaline metal or alkaline-earth metal salt thereof such as a Na, K, Mg or Ca salt and glucosamine will be in free amine form or as an acid addition salt thereof such as the sulfate or the hydrochloride salt. N-acetyl-D-glucosamine is used as such.

Beside the essential components (a)-(d) above, the composition of the present invention may also contain sweetening or flavoring agents destined to the optimization of the organoleptic characteristics of the oral formulation. Said composition may be a pharmaceutical or nutraceutical (as defined in US 5,364,845 and US 5,587,363) composition.

The sweetening agents may be natural, optional reduced sugar such as sucrose, dextrose, xylitol, mannitol or sorbitol, or synthetic product such as sodium saccharine or aspartame. Synthetic sweeteners may be present in a percentage of from 0.1 to 5% while the natural, optional reduced sugar may be present in a percentage of from 10% to 20%, preferably of from 15% to 20%.

The flavoring agents will be chosen by the expert in the field in relation to the organoleptic properties of the composition. The flavoring agents may be selected from the group consisting of pharmaceutically or nutritionally acceptable flavors and tastes of synthetic and natural oils, the latter extracted from plants, leaves, flowers, fruits and their combinations, such as cinnamon, peppermint, anise and citron leaves, bitter almond, citrus fruits, in particular orange and/or lemon, linden and grapefruit oils. Also chocolate, vanilla or eucalyptus flavor and essences of fruit, in particular apple, pear, peach, strawberry, cherry, apricot, orange, lemon and grapes may be advantageously used. Preferred flavoring agents are those giving mint or fruit, such as grape, cherry or citrus, in particular orange and lemon, flavors or mixtures thereof. Flavoring agents, usually supported on a solid matrix, are generally present at a concentration of from 0.5 to 1.5%.

The compositions of the present invention may also contain thickening agents, such as microcrystalline cellulose, hydroxypropylcellulose, carboxymethylcellulose or polyvinylpyrrolidone, in a total amount of from 0.1 % to 0.2%.

The liquid compositions of the present invention are aqueous solutions, preferably in dosage unit forms, each containing
(a) chondroitinsulfate in an amount of from 400 mg to 1500 mg and N-acetylated glucosamine in an amount of from 400 to 1500 mg;
(b) cyclodextrin, which may be natural, such as α-cyclodextrin, β-cyclodextrin and γ-cyclodextrin, or modified, such as hydroxypropyl-β-cyclodextrin, hydroxypropyl-α-cyclodextrin, hydroxypropyl-γ-cyclodextrin, methyl-β-cyclodextrin or sulfobutyl-β-cyclodextrin, in an amount of from 100 to 1000 mg;
(c) antioxidants, such as sodium metabisulfite or sodium sulfite, in a total amount of from 3 to 35 mg; and
(d) preservatives, such as sodium benzoate, sorbic acid and its salts, in particular potassium sorbate, EDTA or a salt thereof, in particular sodium EDTA, *p-*hydroxybenzoic acid esters (parabens), such as methylparaben and ethylparaben, in a total amount of from 2 mg to 20 mg;
in aqueous solution in admixture with common excipients.

The dosage unit preferably consists of an amount of from 4 to 30 g of preferably deionized water containing the components (a)-(d), sweetening and flavoring agents and, optionally, thickening agents.

In said dosage units, the sweetening agents such as those mentioned above may be present in an amount of from 400 mg to 3 g, preferably from 600 mg to 3 g per dosage unit. The flavoring agents may be present in the compositions of the present invention in an amount of from 20 mg to 150 mg of flavoring agent per dosage unit.

The optional thickening agents such as those mentioned above may be present in an amount of from 10 mg to 30 mg per dosage unit.

A particularly advantageous compositions comprises
(a) chondroitinsulfate and N-acetylated glucosamine at a dosage of from 500 mg to 1200 mg and from 500 mg to 1500 mg, respectively;
(b) β-cyclodextrin, hydroxypropyl-β-cyclodextrin or sulfobutyl-β-cyclodextrin;
(c) sodium metabisulfite as antioxidant;
(d) preservatives selected from the group consisting of sodium benzoate, potassium benzoate, potassium sorbate and sodium EDTA;
in a unit dose consisting of an amount of from 4 g to 30 g of aqueous solution containing said components (a)-(d), sweetening agents and flavoring agents.

The unit doses may be in form of welded sachets, little bottles in glass or plastics or any other container rendered available by the current technology, including multidose containers.

The compositions of the present invention may be prepared by the known methods of the pharmaceutical technique.

It is another object of the present invention to provide a method for the preparation of said compositions, which comprises
(A) dissolving the total amount, or at least 50% thereof, of cyclodextrin in water by heating at 35-45°C under stirring to a clear solution, by subsequent cooling of the solution to room temperature and further addition of the preservatives, of the antioxidants and, if any, of the flavoring agents;
(B) adding the N-acetylated glucosamine under stirring to the solution obtained in (A) and kept below 20°C, to a complete solution;
(C) adding a solution, previously prepared by dissolving the optional sweetening agent and chondroitinsulfate in water by heating at 55-65°C under stirring to a clear solution, to the solution obtained in step (B) and, after cooling to room temperature, adding the remaining amount of cyclodextrin, if any, thereto.

If another carrier, for example a thickening agent, is included in the composition, it will be preferably added in step (C).

In general, the total amount of cyclodextrin is used in step (A). It could be advantageous, in the case of the preparation of compositions in which both chondroitinsulfate and N-acetylated glucosamine are present in a small volume of composition (corresponding to 4-7 g), to introduce the cyclodextrin, in particular β-cyclodextrin, in two portions, i.e. about 60% of the calculated amount in step (A) and the remaining amount in step (C).

In the case of higher volumes (corresponding for example to 8-30 g of compositions), it could be advantageous to add a thickening agent such as those mentioned herein above.

The formulations obtainable according to the present invention allow an easy administration even at high combination dosages of chondroitinsulfate and N-acetylated glucosamine in liquid form.

The following examples illustrate the invention without, however, limiting it.

### Comparative example 1

### Solution for the oral administration of 500 mg of chondroitinsulfate and of 500 mg of glucosamine hydrochloride.

(A) In a blender with sleeve and a blade stirrer, 4375 g of hydroxypropyl-β-cyclodextrin and 12 kg of deionized water are added. The temperature is maintained at 40°C and the mixture is vigorously stirred until complete solution. The temperature is brought to 25°C, then 68.75 g of sodium EDTA, 150 g of sodium metabisulfite and 500 g of commercial lemon flavoring agent are added. Stirring is continued at room temperature until a clear solution is obtained.
(B) The temperature of the solution obtained in step (A) is brought to 20°C and 3125 g of glucosamine hydrochloride are showered thereinto. Stirring is continued until a clear solution (Solution B) is obtained.
(C) In a second blender with sleeve and a blade stirrer, 9375 g of xylitol in 15 kg of water are poured, the mixture is heated at 60°C under vigorous stirring to obtain a solution. Under the same stirring and at the same temperature (60°C), 3125 g of sodium chondroitinsulfate are added. The clear solution is then cooled to room temperature, 62.5 g of microcrystalline cellulose (Avicel RC-591) are added and the mixture is stirred until complete dispersion to obtain a solution (Solution C). Solution C thus obtained is added to the previously prepared Solution B under moderate stirring and the resulting solution is passed through a steel sieve 1.5 mm mesh. Thus, about 50 kg of a solution are obtained, to be introduced in 6250 unit doses of the following composition

| | |
|---|---|
| Sodium chondroitinsulfate | 500 mg |
| Glucosamine hydrochloride | 500 mg(*) |
| Hydroxypropyl-β-cyclodextrin | 700 mg |
| Sodium metabisulfite | 24 mg |
| Sodium EDTA | 11 mg |
| Xylitol C | 1500 mg |
| Flavoring agent | 80 mg |
| Microcrystalline cellulose | 10 mg |
| Deionized water to | 8000 mg |

| | |
|---|---|
| (*) corresponding to 416.66 mg of D-glucosamine base. | |

### Comparative example 2

### Solution for the oral administration of 1200 mg of chondroitinsulfate and of 1500 mg ofglucosamine hydrochloride.

The preparation is carried out as described in Example 1 by adding, in step (A), 3182 g of hydroxypropyl-β-cyclodextrin in 12 kg of deionized water, 64 g of sodium EDTA, 145 g of sodium metabisulfite and 455 g of commercial orange flavoring agent. In step (B), 6818 g of glucosamine hydrochloride are added and in step (C) there are added 9090 g of xylitol C, 5454 g of sodium chondroitinsulfate and 72.7 g of microcrystalline cellulose in 15 Kg of deionized water. The resulting solution is passed through a steel sieve 1.5 mm mesh. Thus, about 50 kg of a solution are obtained, to be introduced in 4545 unit doses of the following composition:

| | |
|---|---|
| Sodium chondroitinsulfate | 1200 mg |
| Glucosamine hydrochloride | 1500 mg(*) |
| Hydroxypropyl-β-cyclodextrin | 700 mg |
| Sodium metabisulfite | 32 mg |
| Sodium EDTA | 14 mg |
| Xylitol C | 2000 mg |
| Flavoring agent | 100 mg |
| Microcrystalline cellulose | 16 mg |
| Deionized water to | 11,000 mg |

| | |
|---|---|
| (*) corresponding to 1246.42 mg of D-glucosamine base. | |

### Comparative example 3

### Solution for oral administration of 400 mg of chondroitinsulfate and of 500 mg of glucosamine hydrochloride

(A) In a blender with sleeve and a blade stirrer, 375 g of β-cyclodextrin and 24 kg of deionized water are added. The temperature is maintained at 40°C and the mixture is vigorously stirred until complete solution. The temperature is brought to 25°C, then 110 g of potassium sorbate, 240 g of sodium metabisulfite and 800 g of commercial lemon flavoring agent are added.
(B) The temperature of the solution obtained in step (A) is brought to 20°C and 5000 g of glucosmine hydrochloride are showered thereinto. Stirring is continued until a clear solution (Solution B) is obtained.
(C) In a second blender with sleeve and a blade stirrer, 7500 g of xylitol in 15 kg of water are poured, the mixture is heated at 60°C under vigorous stirring to obtain a solution. Under the same stirring and at the same temperature (60°C), 4000 g of sodium chondroitinsulfate are added. The clear solution is then cooled to room temperature, 250.2 g of β-cyclodextrin are added and the mixture is stirred until complete dispersion to obtain a solution (Solution C). Solution C thus obtained is added to the previously prepared Solution B under moderate stirring and the resulting solution is passed through a steel sieve 1.5 mm mesh. Thus, about 50 kg of a solution are obtained, to be introduced in 10,000 unit doses of the following composition:

| | |
|---|---|
| Sodium chondroitinsulfate | 400.0 mg |
| Glucosamine hydrochloride | 500.0 mg(*) |
| β-cyclodextrin | 65.5 mg |
| Sodium metabisulfite | 24.0 mg |
| Potassium sorbate | 11.0 mg |
| Xylitol C | 750.0 mg |
| Flavoring agent | 80.0 mg |
| Deionized water to | 5000.0 mg |

| | |
|---|---|
| (*) corresponding to 416.66 mg of D-glucosamine base. | |

### Comparative example 4

### Solution for oral administration of 400 mg of chondroitinsulfate and of 500 mg of glucosamine hydrochloride

(A) In a blender with sleeve and a blade stirrer, 375 g of β-cyclodextrin and 24 kg of deionized water are added. The temperature is maintained at 40°C and the mixture is vigorously stirred until complete solution. The temperature is brought to 25°C, then 90 g of sodium benzoate, 20 gr of potassium sorbate, 240 g of sodium metabisulfite and 800 g of commercial lemon flavoring agent are added.
(B) The temperature of the solution obtained in step (A) is brought to 20°C and 5000 g of glucosmine hydrochloride are showered thereinto. Stirring is continued until a clear solution (Solution B) is obtained.
(C) In a second blender with sleeve and a blade stirrer, 7500 g of xylitol in 15 kg of water are poured, the mixture is heated at 60°C under vigorous stirring to obtain a solution. Under the same stirring and at the same temperature (60°C), 4000 g of sodium chondroitinsulfate are added. The clear solution is then cooled to room temperature, 250.2 g of β-cyclodextrin are added and the mixture is stirred until complete dispersion to obtain a solution (Solution C). Solution C thus obtained is added to the previously prepared Solution B under moderate stirring and the resulting solution is passed through a steel sieve 1.5 mm mesh. Thus, about 50 kg of a solution are obtained, to be introduced in 10,000 unit doses of the following composition:

| | |
|---|---|
| Sodium chondroitinsulfate | 400.0 mg |
| Glucosamine hydrochloride | 500.0 mg(*) |
| β-cyclodextrin | 65.5 mg |
| Sodium metabisulfite | 24.0 mg |
| Na Benzoate | 9.0 mg |
| Potassium sorbate | 2.0 mg |
| Xylitol C | 750.0 mg |
| Flavoring agent | 80.0 mg |
| Deionized water to | 5000.0 mg |

| | |
|---|---|
| (*) corresponding to 416.66 mg of D-glucosamine base. | |

### Example 5

### Solution for oral administration of 400 mg of chondroitinsulfate and of 500 mg of N-acetylglucosamine

(A) In a blender with sleeve and a blade stirrer, 375 g of β-cyclodextrin and 24 kg of deionized water are added. The temperature is maintained at 40°C and the mixture is vigorously stirred until complete solution. The temperature is brought to 25°C, then 110 g of potassium sorbate, 240 g of sodium metabisulfite and 800 g of commercial lemon flavoring agent are added.
(B) The temperature of the solution obtained in step (A) is brought to 20°C and 5000 g of N-acetylglucosamine are showered thereinto. Stirring is continued until a clear solution (Solution B) is obtained.
(C) In a second blender with sleeve and a blade stirrer, 7500 g of xylitol in 15 kg of water are poured, the mixture is heated at 60°C under vigorous stirring to obtain a solution. Under the same stirring and at the same temperature (60°C), 4000 g of sodium chondroitinsulfate are added. The clear solution is then cooled to room temperature, 250.2 g of β-cyclodextrin are added and the mixture is stirred until complete dispersion to obtain a solution (Solution C). Solution C thus obtained is added to the previously prepared Solution B under moderate stirring and the resulting solution is passed through a steel sieve 1.5 mm mesh. Thus, about 50 kg of a solution are obtained, to be introduced in 10,000 unit doses of the following composition:

| | |
|---|---|
| Sodium chondroitinsulfate | 400.0 mg |
| N-acetylglucosamine | 500.0 mg |
| β-cyclodextrin | 65.5 mg |
| Sodium metabisulfite | 24.0 mg |
| Potassium sorbate | 11.0 mg |
| Xylitol C | 750.0 mg |
| Flavoring agent | 80.0 mg |
| Deionized water to | 5000.0 mg |

### Example 6

### Solution for oral administration of 400 mg of chondroitinsulfate and of 500 mg of N-acetylglucosamine

(A) In a blender with sleeve and a blade stirrer, 375 g of β-cyclodextrin and 24 kg of deionized water are added. The temperature is maintained at 40°C and the mixture is vigorously stirred until complete solution. The temperature is brought to 25°C, then 90 g of sodium benzoate, 20 gr of potassium sorbate, 240 g of sodium metabisulfite and 800 g of commercial lemon flavoring agent are added.
(B) The temperature of the solution obtained in step (A) is brought to 20°C and 5000 g of N-acetylglucosamine are showered thereinto. Stirring is continued until a clear solution (Solution B) is obtained.
(C) In a second blender with sleeve and a blade stirrer, 7500 g of xylitol in 15 kg of water are poured, the mixture is heated at 60°C under vigorous stirring to obtain a solution. Under the same stirring and at the same temperature (60°C), 4000 g of sodium chondroitinsulfate are added. The clear solution is then cooled to room temperature, 250.2 g of β-cyclodextrin are added and the mixture is stirred until complete dispersion to obtain a solution (Solution C). Solution C thus obtained is added to the previously prepared Solution B under moderate stirring and the resulting solution is passed through a steel sieve 1.5 mm mesh. Thus, about 50 kg of a solution are obtained, to be introduced in 10,000 unit doses of the following composition:

| | |
|---|---|
| Sodium chondroitinsulfate | 400.0 mg |
| N-acetylglucosamine | 500.0 mg |
| β-cyclodextrin | 65.5 mg |
| Sodium metabisulfite | 24.0 mg |
| Na Benzoate | 9.0 mg |
| Potassium sorbate | 2.0 mg |
| Xylitol C | 750.0 mg |
| Flavoring agent | 80.0 mg |
| Deionized water to | 5000.0 mg |

## Claims

1. An oral composition in form of aqueous solution which comprises
(a) chondroitinsulfate at a concentration of from 5% to 15% and N-acetylated glucosamine at a concentration of from 5% to 20% ;
(b) a cyclodextrin at a concentration of from 4% to 10%;
(c) antioxidants at a total concentration of from 0.2% to 0.4%; and
(d) preservatives at a total concentration of from 0.1% to 0.3%.

2. The composition of claim 1, also comprising sweetening agents at a total percent of from 10% to 20% and flavoring agents in a total percent of from 0.05% to 1.5%.

3. The composition of claim 2 which also comprises thickening agents at a total percent of from 0.1% to 0.2%.

4. The composition of anyone of claims 1-3, which is in dosage unit form.

5. The composition of anyone of claims 1-3, which is in a dosage unit form consisting of from 4 g to 30 g of aqueous solution of the components (a)-(d).

6. The composition of claim 5, wherein said dosage unit form comprises
(a) chondroitinsulfate in an amount of from 400 to 1500 mg and N-acetylated glucosamine in an amount of from 400 mg to 1500 mg;
(b) cyclodextrin in an amount of from 200 mg to 1000 mg;
(c) antioxidants in a total amount of from 3 mg to 35 mg; and
(d) preservatives in a total amount of from 2 mg to 20 mg.

7. The composition of claim 6, wherein said cyclodextrin is β-cyclodextrin, hydroxypropyl-β-cyclodextrin or sulfobutyl-β-cyclodextrin.

8. The composition of claim 6, wherein said antioxidant is sodium metabisulfite o sodium sulfite.

9. The composition of claim 6, wherein said preservatives are selected from the group consisting of sodium benzoate, sorbic acid and their salts, EDTA and their salts and *p-*hydroxybenzoic acid esters.

10. The composition of claim 6, also comprising sweetening agents in a total amount of from 400 mg to 3 g and flavoring agents in a total amount of from 20 mg to 150 mg.

11. The composition of claim 6, wherein
(a) chondroitinsulfate and N-acetylated glucosamine are present in an amount of from 500 to 1200 mg and from 400 to 1500 mg, respectively;
(b) the cyclodextrin is β-cyclodextrin, hydroxypropyl-β-cyclodextrin or sulfobutyl-β-cyclodextrin;
(c) the antioxidant is sodium metabisulfite;
(d) the preservatives are selected from the group consisting of sodium benzoate, potassium sorbate and sodium EDTA;
in a dosage unit form consisting of an amount of from 4 to 30 g of solution containing the components (a)-(d), sweetening agents and flavoring agents.

12. The composition of claim 11, wherein the sweetening agents are selected from the group consisting of xylitol, sorbitol and mannitol in an amount of from 400 mg to 3 g per dosage unit.

13. The composition of claim 11, wherein the flavoring agents are present in an amount of from 5 mg to 150 mg per dosage unit.

14. The composition of claim 11 also comprising a thickening agent selected from the group consisting of microcrystalline cellulose, hydroxypropylcellulose, carboxymethylcellulose and polyvinylpyrrolidone in an amount of from 10 mg to 30 mg per dosage unit.

15. A method for the preparation of the composition of anyone of claims 1-3, which comprises
(A) dissolving the total amount, or at least 50% thereof, of cyclodextrin in water by heating at 35-45°C under stirring to a clear solution, cooling of the solution to room temperature and adding the preservatives, the antioxidants and the flavoring agents, if any;
(B) adding the N-acetylated glucosamine under stirring to the solution obtained in (A) and kept below 20°C, to a complete solution;
(C) adding a solution, previously prepared by dissolving the optional sweetening agent and chondroitinsulfate in water by heating at 55-65°C under stirring to a clear solution, to the solution obtained in step (B) and, after cooling to room temperature, adding the remaining amount of cyclodextrin, thereto.

## Patentansprüche

1. Eine orale Zusammensetzung in Form einer wässrigen Lösung, die umfasst:
(a) Chondroitinsulfat in einer Konzentration von 5% bis 15% und N-acetyliertes Glucosamin in einer Konzentration von 5% bis 20%;
(b) ein Cyclodextrin in einer Konzentration von 4% bis 10%;
(c) Antioxidationsmittel in einer Gesamtkonzentration von 0,2% bis 0,4%; und
(d) Konservierungsmittel in einer Gesamtkonzentration von 0,1% bis 0,3%.

2. Die Zusammensetzung gemäß Anspruch 1, weiterhin umfassend Süßungsmittel in einer Gesamtkonzentration von 10% bis 20% und Geschmacksstoffe in einer Gesamtkonzentration von 0,05% bis 1,5%.

3. Die Zusammensetzung gemäß Anspruch 2, die weiterhin Verdickungsmittel in einer Gesamtkonzentration von 0,1 % bis 0,2% aufweist.

4. Die Zusammensetzung gemäß irgendeinem der Ansprüche 1 bis 3, die in Form von Dosiereinheiten vorliegt.

5. Die Zusammensetzung gemäß irgendeinem der Ansprüche 1 bis 3, die in Form von Dosiereinheiten vorliegt, bestehend aus 4 g bis 30 g einer wässrigen Lösung der Komponenten (a) bis (d).

6. Die Zusammensetzung gemäß Anspruch 5, wobei die Dosiereinheit umfasst
(a) Chondroitinsulfat in einer Menge von 400 bis 1500 mg und N-acetyliertes Glucosamin in einer Menge von 400 mg bis 1500 mg;
(b) Cyclodextrin in einer Menge von 200 mg bis 1000 mg;
(c) Antioxidationsmittel in einer Gesamtmenge von 3 mg bis 35 mg; und
(d) Konservierungsmittel in einer Gesamtmenge von 2 mg bis 20 mg.

7. Die Zusammensetzung gemäß Anspruch 6, wobei das Cyclodextrin β-Cyclodextrin, Hydroxypropyl-β-Cyclodextrin oder Sulfobutyl-β-Cyclodextrin ist.

8. Die Zusammensetzung gemäß Anspruch 6, wobei das Antioxidationsmittel Natriummetabisulfit oder Natriumsulfit ist.

9. Die Zusammensetzung gemäß Anspruch 6, wobei die Konservierungsmittel ausgewählt sind aus der Gruppe bestehend aus Natriumbenzoat, Sorbinsäure und ihren Salzen, EDTA und deren Salze und p-Hydroxybenzoesäureester.

10. Die Zusammensetzung gemäß Anspruch 6, weiterhin umfassend Süßungsmittel in einer Gesamtmenge von 400 mg bis 3 g und Geschmacksstoffe in einer Gesamtmenge von 20 mg bis 150 mg.

11. Die Zusammensetzung gemäß Anspruch 6, wobei
(a) Chondroitinsulfat und N-acetyliertes Glucosamin in einer Menge von 500 bis 1200 mg beziehungsweise von 400 bis 1500 mg vorhanden sind;
(b) das Cyclodextrin β-Cyclodextrin, Hydroxypropyl-β-Cyclodextrin oder Sulfobutyl-β-Cyclodextrin ist;
(c) das Antioxidationsmittel Natriummetabisulfit ist;
(d) die Konservierungsmittel ausgewählt sind aus der Gruppe bestehend aus Natriumbenzoat, Kaliumsorbat und Natrium-EDTA;
in einer Dosiereinheit bestehend aus einer Menge von 4 bis 30 g einer Lösung, die die Komponenten (a) bis (d), Süßungsmittel und Geschmacksstoffe enthält.

12. Die Zusammensetzung gemäß Anspruch 11, wobei die Süßungsmittel ausgewählt sind aus der Gruppe bestehend aus Xylit, Sorbit und Mannit in einer Menge von 400 mg bis 3 g pro Dosiereinheit.

13. Die Zusammensetzung gemäß Anspruch 11, wobei die Geschmacksstoffe in einer Menge von 5 mg bis 150 mg pro Dosiereinheit vorliegen.

14. Die Zusammensetzung gemäß Anspruch 11, weiterhin enthaltend ein Verdickungsmittel ausgewählt aus der Gruppe bestehend aus mikrokristalliner Cellulose, Hydroxypropylcellulose, Carboxymethylcellulose und Polyvinylpyrrolidon in einer Menge von 10 mg bis 30 mg pro Dosiereinheit.

15. Verfahren zur Herstellung der Zusammensetzung nach irgendeinem der Ansprüche 1 bis 3, umfassend
(A) Auflösen der Gesamtmenge, oder mindestens 50% davon, von Cyclodextrin in Wasser durch Erhitzen bei 35 bis 45°C unter Rühren zu einer klaren Lösung, Abkühlen der Lösung auf Raumtemperatur und Zugeben der Konservierungsmittel, der Antioxidantien und Geschmacksmittel, falls vorhanden;
(B) Zugeben des N-acetylierten Glucosamins unter Rühren zu der in (A) erhaltenen Lösung und Halten unter 20°C, zu einer vollständigen Lösung;
(C) Zugeben einer Lösung, die zuvor durch Lösen des optionalen Süßungsmittels und des Chondroitinsulfats in Wasser mittels Erhitzen bei 55 bis 65 °C unter Rühren zu einer klaren Lösung erhalten wurde, zu der in Schritt (B) erhaltenen Lösung, und nach dem Abkühlen auf Raumtemperatur, Zugeben der restlichen Menge von Cyclodextrin dazu.

## Revendications

1. Composition orale sous la forme d'une solution aqueuse qui comprend
(a) une chondroïtine sulfate à une concentration de 5 % à 15 % et une glucosamine N-acétylée à une concentration de 5 % à 20 % ;
(b) une cyclodextrine à une concentration de 4 % à 10 % ;
(c) des antioxydants à une concentration totale de 0,2 % à 0,4 % ; et
(d) des conservateurs à une concentration totale de 0,1 % à 0,3 %.

2. Composition selon la revendication 1, comprenant également des agents édulcorants à un pourcentage total de 10 % à 20 % et des agents aromatisants à un pourcentage total de 0,05 % à 1,5 %.

3. Composition selon la revendication 2, qui comprend également des agents épaississants à un pourcentage total de 0,1 % à 0,2 %.

4. Composition selon l'une quelconque des revendications 1 à 3, qui est sous une forme d'unité de dosage.

5. Composition selon l'une quelconque des revendications 1 à 3, qui est sous une forme d'unité de dosage constituée de 4 g à 30 g d'une solution aqueuse des composants (a) à (d).

6. Composition selon la revendication 5, dans laquelle ladite forme d'unité de dosage comprend
(a) une chondroïtine sulfate en une quantité de 400 à 1500 mg et une glucosamine N-acétylée en une quantité de 400 mg à 1500 mg ;
(b) une cyclodextrine en une quantité de 200 mg à 1000 mg ;
(c) des antioxydants en une quantité totale de 3 mg à 35 mg ; et
(d) des conservateurs en une quantité totale de 2 mg à 20 mg.

7. Composition selon la revendication 6, dans laquelle ladite cyclodextrine est la β-cyclodextrine, l'hydroxypropyl-β-cyclodextrine ou la sulfobutyl-β-cyclodextrine.

8. Composition selon la revendication 6, dans laquelle ledit antioxydant est le métabisulfite de sodium ou le sulfite de sodium.

9. Composition selon la revendication 6, dans laquelle lesdits conservateurs sont choisis dans le groupe constitué par le benzoate de sodium, l'acide sorbique et ses sels, l'EDTA et ses sels et les esters d'acide p-hydroxybenzoïque.

10. Composition selon la revendication 6, comprenant également des agents édulcorants en une quantité totale de 400 mg à 3 g et des agents aromatisants en une quantité totale de 20 mg à 150 mg.

11. Composition selon la revendication 6, dans laquelle
(a) la chondroïtine sulfate et la glucosamine N-acétylée sont présentes en une quantité de 500 à 1200 mg et de 400 à 1500 mg, respectivement ;
(b) la cyclodextrine est la β-cyclodextrine, l'hydroxypropyl-β-cyclodextrine ou la sulfobutyl-β-cyclodextrine ;
(c) l'antioxydant est le métabisulfite de sodium ;
(d) les conservateurs sont choisis dans le groupe constitué par le benzoate de sodium, le sorbate de potassium et l'EDTA sodique ;
sous une forme d'unité de dosage constituée d'une quantité de 4 à 30 g d'une solution contenant les composants (a) à (d), des agents édulcorants et des agents aromatisants.

12. Composition selon la revendication 11, dans laquelle les agents aromatisants sont choisis dans le groupe constitué par le xylitol, le sorbitol et le mannitol en une quantité de 400 mg à 3 g par unité de dosage.

13. Composition selon la revendication 11, dans laquelle les agents aromatisants sont présents en une quantité de 5 mg à 150 mg par unité de dosage.

14. Composition selon la revendication 11, comprenant également un agent épaississant choisi dans le groupe constitué par la cellulose microcristalline, l'hydroxypropylcellulose, la carboxyméthylcellulose et la polyvinylpyrrolidone en une quantité de 10 mg à 30 mg par unité de dosage.

15. Procédé de préparation de la composition selon l'une quelconque des revendications 1 à 3, qui comprend
(A) la dissolution de la quantité totale, ou d'au moins 50 % de celle-ci, de cyclodextrine dans de l'eau en chauffant à une température de 35 à 45 °C sous agitation jusqu'à l'obtention d'une solution limpide, le refroidissement de la solution jusqu'à la température ambiante et l'ajout des conservateurs, des antioxydants et des agents aromatisants, s'il y en a ;
(B) l'ajout de la glucosamine N-acétylée sous agitation à la solution obtenue en (A) et maintenue sous 20 °C, pour obtenir une solution complète ;
(C) l'ajout d'une solution, préalablement préparée par la dissolution de l'agent édulcorant facultatif et de la chondroïtine sulfate dans de l'eau en chauffant à une température de 55 à 65 °C sous agitation jusqu'à l'obtention d'une solution limpide, à la solution obtenue dans l'étape (B) et, après refroidissement jusqu'à la température ambiante, l'ajout de la quantité restante de cyclodextrine à celle-ci.
